(19) Europäisches Patentamt
European Patent Office
Office européen des brevets

(11) **EP 3 556 420 B1**

(12) **EUROPEAN PATENT SPECIFICATION**

(45) Date of publication and mention of the grant of the patent:
**09.12.2020 Bulletin 2020/50**

(51) Int Cl.:
*A61M 16/12* *(2006.01)*        *B01F 3/02* *(2006.01)*
*A61M 16/10* *(2006.01)*

(21) Application number: **19167389.6**

(22) Date of filing: **04.04.2019**

(54) **GAS MIXER FOR PROVING A GAS MIXTURE TO A MECHANICAL VENTILATOR**

GASMISCHER ZUR PRÜFUNG EINES GASGEMISCHES AN EINEN MECHANISCHEN VENTILATOR

MÉLANGEUR DE GAZ POUR FOURNIR UN MÉLANGE GAZEUX À UN VENTILATEUR MÉCANIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.04.2018 US 201862660303 P**

(43) Date of publication of application:
**23.10.2019 Bulletin 2019/43**

(73) Proprietor: **L'AIR LIQUIDE, SOCIETE ANONYME POUR L'ETUDE ET L'EXPLOITATION DES PROCEDES GEORGES CLAUDE**
**75007 Paris (FR)**

(72) Inventor: **BOULANGER, Thierry**
**Newark, Delaware 19702 (US)**

(74) Representative: **Air Liquide**
**L'Air Liquide S.A.**
**Direction de la Propriété Intellectuelle**
**75, Quai d'Orsay**
**75321 Paris Cedex 07 (FR)**

(56) References cited:
**EP-A1- 2 489 392**        **WO-A2-2015/037002**
**US-A1- 2009 205 661**        **US-A1- 2014 144 440**

## Description

### Background

The present invention relates to a gas mixing device or mixer that can be used in hospitals for operating precise deliveries of various gas mixtures, especially when fluidly connected to a medical ventilator.

Gas mixtures are commonly used for treating various respiratory deficiencies or diseases affecting different populations of patients.

For instance, oxygen-enriched air is often used for treating hypoxemic conditions. Such an oxygen/air mixture can be obtained in situ, typically directly in the nostrils of the patient, by mixing an $O_2$ flow delivered by a nasal cannula, at a continuous flowrate of between 1 and 6 L/min, with ambient air inspired by the patient during inspiratory phases.

In case of a severe respiratory distress, such as COPD exacerbation affecting adult patients or acute asthma attacks affecting pediatric patients, at least a part of the nitrogen molecules ($N_2$) contained in air can be replaced by helium (He) to decrease the breathing work of the patients. This is typically done by means of a gas mixing device, also called "mixer" or "blender", which is connected to several sources of gas, such as $O_2$ and He. The $O_2$ concentration, i.e. the He/$O_2$ ratio, is generally adjusted by the medical staff as it depends on the patient's needs.

Medical gases are also used as therapeutic agents for treating other medical conditions or diseases. For instance, pain and anxiety can be relieved by inhaling a gaseous mixture of $O_2$ and nitrous oxide ($N_2O$), whereas a ternary mixture containing 50 vol.% argon (Ar), 21 vol.% $O_2$ and 29% $N_2$ (vol.%) has shown neuroprotective properties.

Despite the fact that gas mixtures are widely used for treating patients, especially in hospitals or the like, delivering a hypoxic mixture to the patient is a constant risk and it is important to make sure the concentration of oxygen in the gas mixture administrated to the patients is never unintentionally less than 21 vol.%.

The gas administration has hence to be controlled both in operation and in case of failure of the gas mixing device.

Architectures of gas mixers have been proposed by US-A-2008/0121233, US-A-2009/0205661 and EP-A-2489392.

For instance, EP-A-2489392 discloses a gas mixing device (blender) including a pressure vessel of about 0.5 L which stores the gas mixture above the atmospheric pressure, for example at 2 bar abs. An on-demand valve is arranged between the pressure vessel and the patient. This valve opens only when a pressure decrease occurs (due to an inhalation phase of the patient) and a part of the gas mixture of the vessel is then delivered to the patient. The vessel is refilled when the gas pressure measured in said vessel drops below a given threshold value.

However, with existing blenders, it can be difficult to realize a precise control of the concentrations in the gas mixture, especially the oxygen concentration, and further to detect hypoxic situations that could be risky for the patient.

### Summary

A goal of the present invention is to provide a gas mixer able to deliver a concentration of up to three gases, such as a therapeutic gas, oxygen and air, that overcomes at least part of the drawbacks of the blenders of the prior art.

A solution according to the present invention concerns a gas mixer comprising:

- a mixing vessel,
- a first line for providing a first gas, a second line for providing a second gas and a third line for providing a third gas, said first, second and third lines being in fluid communication with the mixing vessel for proving said first, second and third gases to said mixing vessel and obtaining a gas mixture in said mixing vessel, and
- a delivery line in fluid communication with the mixing vessel for recovering at least a part of the gas mixture contained in the mixing vessel,

and wherein:

- the first line comprises a first pressure regulator, a first pressure sensor and a first mass flow controller comprising a first proportional valve and a first mass flow sensor,
- the second line comprises a second pressure regulator, a second pressure sensor and a second mass flow controller comprising a second proportional valve and a second mass flow sensor,
- the third line comprises a third pressure regulator, a third pressure sensor and a third mass flow controller comprising a third proportional valve and a third mass flow sensor,
- the first, the second and the third lines are in fluid communication with the mixing vessel via an admission line, and
- an oxygen sensor is arranged on the admission line.

Depending on the embodiment, a gas mixer according to the present invention can comprise one or several of the following features:

- the admission line is in fluid communication with the first, second and third lines and with the mixing vessel.
- the admission line is in fluid communication with the second and third lines via a first common line.
- a fourth mass flow sensor is arranged on the first common line.
- a fifth mass flow sensor is arranged on the admission line between the oxygen sensor and the mixing vessel.
- the mixing vessel is further fluidly connected to a delivery line for withdrawing and conveying at least a part of the gaseous mixture contained in said mixing vessel.
- the delivery line comprises a fourth proportional valve.
- the delivery line further comprises a fourth pressure sensor arranged downstream of the fourth proportional valve.
- the mixer further comprises a control unit.
- the first line is fluidly connected to a first gas source, the second line is fluidly connected to a second gas source and the third line is fluidly connected to a third gas source.
- the first gas source delivers air.
- the second gas source delivers oxygen gas.
- the third gas source delivers a therapeutically-effective gas.
- the therapeutically-effective gas is chosen among $N_2O$, argon, xenon, helium or nitrogen.
- the mixer comprises a setting interface connected to the control unit.
- the control unit is configured for processing pressure signals received from the pressure sensors and from the mass flow sensors.
- the control unit is further configured for processing oxygen concentration signals received from the oxygen sensor.
- the control unit is further configured for controlling the proportional valves.

Further, the invention also concerns a mechanical ventilation system comprising gas mixer according to the present invention, in fluid communication with a mechanical ventilator for providing a gas mixture to said mechanical ventilator.

## Brief Description of the Drawings

The present invention will be explained in more details in the following illustrative description of an embodiment of a gas mixing device or mixer according to the present invention, which is made in references to the accompanying drawings among them:

- Figure 1 shows an embodiment of the architecture of a gas mixer according to the present invention,
- Figure 2 shows a gas mixer cooperating with a mechanical ventilator, and
- Figure 3 shows a second embodiment of a mixer according to the present invention that is able to operate with a mechanical ventilator.

## Description of the Preferred Embodiments

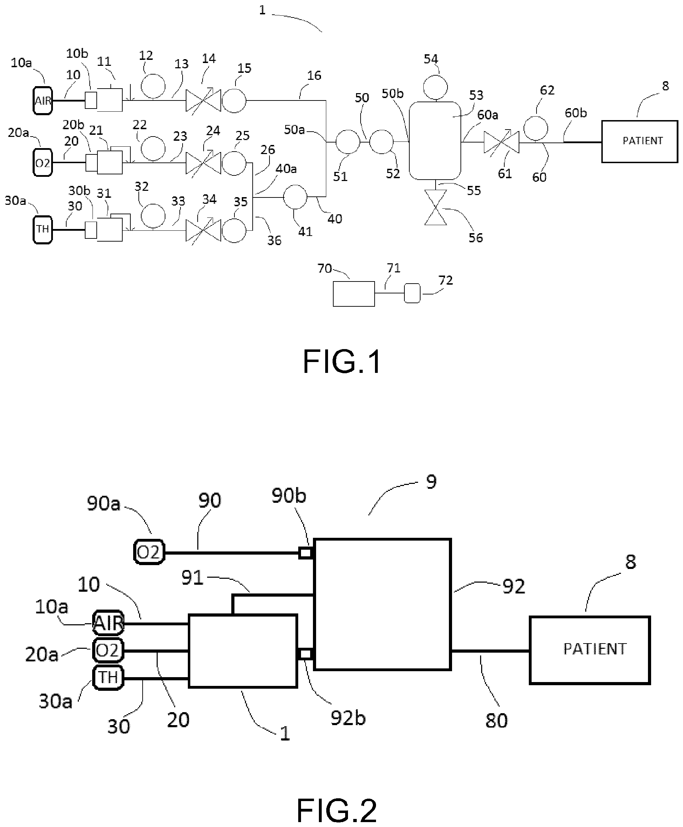

In Figure 1, a first embodiment of a gas mixer **1** according to the present invention, that is fluidly connected to a patient **8** at the end **60b** of a delivery line **60,** is shown.

The mixer **1** of Figure 1 comprises a first, a second and a third gas line **13, 23, 33,** respectively, comprising a first, a second and a third inlet port **10b, 20b, 30b,** respectively, wherein:

- the first inlet port **10b** is fluidly connected, via a first inlet line **10,** to a first source **10a** of a first gas, namely a medicinal air source, for instance an air delivery plug arranged on a wall and fed by the gas network of a hospital,
- the second port **20b** is fluidly connected, via a second inlet line **20,** to a second source **20a** of a second gas, namely a medical $O_2$ source **20a,** for instance an oxygen delivery plug also fed by the gas network of the hospital,
- the third port **30b** is fluidly connected, via a third inlet line **30,** to a third source **30a** of a third gas, namely a therapeutically-effective gas (TH) source **30a,** preferably a gas cylinder or the like. For instance, the therapeutically-effective gas can be $N_2O$, argon, xenon, helium or nitrogen.

The first, second and third gas lines **13, 23, 33** are arranged in parallel.

The mixer **1** comprises different electronic elements, such as mass flow controllers and sensors, that are controlled by a control unit **70,** whose core component is a microprocessor or a microcontroller.

The control unit **70** further provides electric power to the electronic elements and is able to read and process the measurements provided by the sensors, typically signals delivered by the sensors.

The control unit **70** embeds conversion lookup tables or the like, for determining a flow measurement based on the nature of the gas being measured, here for instance medicinal air, $O_2$, therapeutic gas and combinations thereof.

The control unit **70** is further in communication, via electric line **71,** to a command unit **72** operable by a user for setting the concentrations of the different gases in the mixture to be realized in vessel **53,** which is afterwards delivered to the patient **8** via delivery line **60.**

First line **13** comprises, in series, downstream of the first inlet port **10b,** a first pressure regulator **11,** a first pressure sensor **12** and a first mass flow controller **14, 15.** The role of each of those elements is detailed hereafter.

First pressure regulator **11** is provided for reducing the pressure of the first gas, e.g. air, delivered by the first gas source, e.g. a medicinal air source **10a.** The level of the desired lower pressure depends on the settings of the first pressure regulator **11.** For instance, if the pressure of the first gas is of about 3.5 bars abs, the pressure level of the first pressure regulator **11** can be set to a lower pressure of 2.5 bars abs (of course, it could be higher or lower) so that the first gas exhibits said lower pressure downstream of the first pressure regulator **11,** in first line **13.**

First pressure sensor **12,** that is in communication with control unit **70,** is used for monitoring the gas pressure in first line **13,** downstream of the first pressure regulator **11,** to make sure that the pressure value of the first gas is within acceptable ranges, for instance +/- %5 with respect to a given pressure value. First pressure sensor **12** can also indicate to control unit **70** that no or insufficient pressure exists in first line **13,** which means that the first source **10a,** such as a medicinal air source, is not connected to the first inlet port **10b.**

First mass flow controller **14, 15** comprises a first proportional valve **14** and a first mass flow sensor **15.** The first proportional valve **14** is controlled by control unit **70** which determines its opening status (i.e. valve more or less open) based on a targeted flow measured by the first mass flow sensor **15,** whereas first mass flow sensor **15** is configured by a lookup table relating to the first gas, such as medicinal air. The gaseous flow passing through first proportional valve **14** travels first into first mass flow sensor **15** and then in the downstream portion **16** of first line **13.**

Similarly, the second line **23** comprises, in series, downstream of the second inlet port **20b** receiving the second gas from the second source **20a,** e.g. a medicinal $O_2$ source, a second pressure regulator **21,** a second pressure sensor **22** and a second mass flow controller **24, 25** comprising a second proportional valve **24** and a second mass flow sensor **25,** whereas the third line **33** also comprises, in series, downstream of the third inlet port **30b** receiving the third gas from the third source **30a,** e.g. a therapeutically-effective gas source, a third pressure regulator **31,** a third pressure sensor **32** and a third mass flow controller **34, 35** comprising a third proportional valve **34** and a third mass flow sensor **35.** As all those elements run and/or are operated the same way as the corresponding ones arranged on the first line **13,** they will not be detailed further.

The mixer **1** provides the ability to deliver up to 3 gases coming from the first, second and third gas sources **10a, 20a, 30a.**

As the first, second and third pressure sensors **12, 22** and **32** inform the control unit **70** of the presence of gas sources fluidly connected to the first, second and third ports **10b, 20b, 30b,** some specific gas mixtures can be prohibited for increasing the safety for the patient **8.** This means that the control unit **70** can be configured for not authorizing the delivery of some specific mixtures to patient **8.**

For instance, control unit **70** can be configured for

- allowing the delivery of gas if it is detected that second gas source **20a** (e.g. medical $O_2$) and third gas source **30a** (e.g. therapeutic gas) and/or first gas source **10a** (e.g. medicinal air) are simultaneously connected to their respective ports **10b, 20b, 30b,** or
- in contrast, prohibiting the delivery of gas if it is detected that the third gas source **30a** (e.g. therapeutic gas) is the only source connected or that the first gas source **10a** (e.g. medicinal air) is the only additional source simultaneously connected with third gas source **30a.**

In other words, thanks to the control unit **70** that independently pilots the first, second and third proportional valves **14, 24, 34,** the mixer **1** of the present invention is able to deliver a mixture of only two gases, for instance a mixture of 50 vol.% $O_2$ and 50 vol.% $N_2O$ as the therapeutic gas, or three gases, for instance a mixture of 50 vol.% Ar as the therapeutic gas, 21% $O_2$ and the rest being $N_2$; of course, other amounts of gas can be delivered.

As shown in Figure 1, the downstream portions **26, 36** of second and third gas lines **23, 33** are fluidly connected to a first common line **40,** i.e. they are branched at a first location **40a,** so that gases coming from the second and third gas lines **23, 33** can travel in said first common line **40** toward vessel **53.**

In the same way, common line **40** and downstream portion **16** of first gas line **13** are fluidly connected to a second common line **50,** also called admission line, i.e. they are branched at second location **50a,** so that gases coming from the first gas line **13** and from the first common line **40** (that receives gas from the second and third gas lines **23, 33**) can travel in said admission line **50,** toward vessel **53.**

Admission line **50** or second common line is fluidly connected to pressure vessel **53** via an inlet orifice **50b,** for delivering the different gases to be mixed in said vessel **53.**

The pressurized vessel **53** has a suitable volume typically comprised between 0.1 and 5 L, for example a volume of about 0.5 L, wherein the gas mixture is stored at a desired pressure or in a desired pressure range, for instance between 2 and 2.5 bars abs. Vessel **53** constitutes a mixing chamber for the gases.

Mixing vessel **53** is further fluidly connected to the upstream end **60a** of a delivery line **60** which conveys the gaseous mixture withdrawn from vessel **53** to the patient **8.** Delivery line **60** comprises a fourth proportional valve **61** and a fourth pressure sensor **62,** the latter being arranged downstream of the fourth proportional valve **61.**

Control unit **70** receives pressure measurement signals from the fourth pressure sensor **62** and further controls said fourth proportional valve **61** based on said pressure measurement signals.

The downstream portion **60b** of delivery line **60** delivers the gaseous mixture to the patient **8,** for instance by means of a respiratory interface, such as a respiratory mask or the like.

When a patient **8** is connected to terminal downstream end **60b** of delivery line **60** and starts to inhale gas, the pressure downstream of proportional valve **61** falls below the atmospheric pressure, which will be measured/detected by the fourth pressure sensor **62.**

In response to that pressure decreasing, control unit **70** that controls the fourth proportional valve **61** sets the opening status of the fourth proportional valve **61** to counterbalance the pressure decreasing measured by the fourth pressure sensor **62,** that reflects the patient's flow demand. A part of the gas mixture stored in vessel **53** is then drawn into delivery line **60,** whereas the pressure in vessel **53** decreases. Said pressure decreasing is detected by vessel pressure sensor **54** that is arranged on vessel **53** so as to monitor the pressure variations in pressure vessel **53.**

Measurement signals delivered by vessel pressure sensor **54** are recovered by control unit **70** that is also electrically connected to vessel pressure sensor **54.**

In response to that pressure decrease in vessel **53,** control unit **70** actuates first, second and third proportional valves **14, 24, 34** so as to allow a passage of the different gases through said valves **14, 24, 34.** The pressurized gases travel in the different lines **16, 26, 36, 40** and admission line **50** before reaching vessel **53** thereby refilling vessel **53** with the right gas mixture until the pressure in said vessel **53,** measured by vessel pressure sensor **54,** reaches again the desired pressure range, for instance between 2 and 2.5 bars abs.

According to the present invention, it is also provided safety elements for preventing hypoxic situations, precisely controlling the gas mixture delivered to the patient **8** and monitoring the concentration of the gas mixture realized in vessel **53.**

More precisely, according to the present invention, it is operated a pre-use verification sequence and subsequently a real-time monitoring of the mixer status to guarantee a safe and precise operation.

**Pre-use verification sequence**

The pre-use verification sequence is initiated by the control unit **70** in response to a command input by the operator operated on the command unit **72** that is connected to the control unit **70** by means of a connecting line **71.**

Upon initiation of the pre-use verification, control unit **70** shuts the first, second and third proportional valves **14, 24** and **34** so that no gas can travel in the first, second and third lines **16, 26** and **36.**

Meanwhile, control unit **70** shuts the fourth proportional valve **61** to prevent gas travel into delivery line **60,** in the case where a patient **8** is already connected to line **60,** and opens a dump valve **56** which is in fluid communication with the inner volume of pressure vessel **53,** via a venting line **55.** Dump valve **56,** when opened, allows the gas contained into vessel **53** to be vented to the atmosphere.

After this sequence is performed, control unit **70** opens the third proportional valve **34** so that a flow of about 1 L/min, measured by the third mass flow sensor **35** travels into third line **36** and first common line **40.** Line **40** comprises a fourth mass flow sensor **41.**

At this stage, control unit **70** configures the fourth mass flow sensor **41** with a lookup table corresponding to the first gas source **30a** containing the therapeutically-effective gas.

A first verification step made by control unit **70** comprises a step of confirming that third and fourth mass flow sensors **35, 41** measure about the same flow of gas, which significates that those third and fourth mass flow sensors **35, 41** are operational.

In admission line **50,** the flow of gas then travels into two additional sensors comprising an oxygen sensor **51** and a fifth mass flow sensor **52.**

Oxygen sensor **51** is for instance a zirconia based sensor, such as the one referenced KGZ-10 commercialized by the Honeywell Company, which is designed for operating at pressures up to 3 bar abs. Such a sensor is linear while operating, meaning that its output is linearly dependent on the oxygen concentration (i.e. from 0 to 100%).

At this stage, the flow of gas in contact with the oxygen sensor **51** comes from the therapeutic gas source **30a** which contains 0% of $O_2$. This characteristic helps set a first calibration point for the oxygen sensor **51** by measuring the output of said sensor **51** via the control unit **70** and determine that said output corresponds to a 0% point (also called zeroing point).

In a same way, the fifth mass-flow sensor **52,** configured by control unit **70** with respect to the properties of therapeutic gas source **30a,** measures a flow equal to those measured by mass flow sensors **35, 41,** further confirming its correct operation.

The flow of gas exiting admission line **50** enters vessel **53** and is vented to the atmosphere via dump valve **56** as above explained.

Once this sequence completed, the control unit **70** switches the gas sources. First proportional valve **14** is kept in its close position and third proportional valve **34** is closed, whereas second proportional valve **24** is opened so that a flow of $O_2$ of about 1 L/min, measured by the second mass flow sensor **25** is traveling successively into second line **26** and common line **40** that comprises the fourth mass flow sensor **41**.

At this stage, control unit **70** configures the fourth mass flow sensor **41** with a lookup table corresponding to the second gas, typically medical $O_2$ gas, coming from the second gas source **20a.**

As already explained, it is operated another verification step for confirming that the third and fourth mass flow sensors **35, 41** measure about the same flow of oxygen, for ensuring that the second mass flow sensor **35** is also operational.

The flow of oxygen travels into admission line **50,** where it encounters oxygen sensor **51** and the fifth mass flow sensor **52**. The flow in contact with the oxygen sensor **51** is the second gas, namely $O_2$ gas (purity of at least 99.5 vol.%). This leads to a second calibration point for the oxygen sensor **51** by measuring the output of said sensor **51** via control unit **70** as said output corresponds to a 100% point (also called full scale point).

As the control unit **70** has determined the 0% and the 100% of $O_2$ points, thanks to the calibration steps, it is able to precisely evaluate the oxygen concentration in contact with the oxygen sensor **51** by applying a linear relationship between the output of said sensor **51** and the actual content of a given gas mixture.

The oxygen flow can then enter into vessel **53** and be vented to the atmosphere via dump valve **56** as already explained.

At this stage, control unit **70** of mixer **1** has determined that second, third, fourth and fifth mass flow sensors **25, 35, 41, 52** are correctly operating, and that oxygen sensor **52** is fully calibrated.

The last step of verification consists in closing the second and third proportional valves **24, 34** and opening the first proportional valve **14** so that a flow of 1 L/min, measured by the fist mass flow sensor **15,** can travel into the first line **16**.

Then, when proceeding as above explained for the other gases, control unit **70** is able to determine that the oxygen concentration of the first gas delivered by the first gas source **10a,** namely medicinal air, contains about between 19.5% and 23.5%, of oxygen (according to the US Pharmacopoeia).

The output of oxygen sensor **51** is then stored by control unit **70**. Configuring first and fifth mass flow sensors **15, 52** with a lookup table corresponding to medicinal air (the variation of the oxygen concentration into nitrogen has no impact on the measurement for mass flow sensors), it can be determined that the first mass flow sensor **15** operates correctly. Again, the flow travels to vessel **53** and is afterwards vented by dump valve **56.**

This last sequence closes the pre-use verification sequence and, unless a discrepancy is found, for example mass flow sensors measurements differences, the mixer **1** is considered as being ready for operation.

**Real-time Monitoring**

Operation of mixer **1** starts when patient **8** is connected to delivery line **60** and the operator has set a gaseous mixture to be delivered to said patient **8,** by means the command unit **72,** such as a keyboard or similar.

As an example, a ternary mixture consisting of 50 vol.% Ar (third source **30a**), 21 vol.% of $O_2$ (second source **20a**) and 29 vol.% of air containing about 80 vol.% of $N_2$ (first source **10a**) is selected.

As described above, the refilling of vessel **53** depends on the patient **8** demand based on the measurement of vessel pressure sensor **54** (to keep the pressure in said vessel **53** within an acceptable range, such as between 2 and 2.5 bar abs).

Whenever there is a need to refill vessel **53,** control unit **70** sets different flow targets to the first, second and third proportional valves **14, 24, 34** based on the measurement of the first, second and third mass flow sensors **15, 25** and **35,** which are naturally configured through proper lookup tables with respect to the gas source they are connected to.

The gaseous flows traveling in each of the first, second and third lines **16, 26, 36** are a directly related to the concentrations set by the user. Assuming for instance a total flow $Q_{TOT}$ traveling into admission line **50** to refill vessel **53,** it can be determined that flows $Q_{AR}$ (travelling into third line **36**), $Q_{O2}$ (travelling into second line **26**) and $Q_{AIR}$ (travelling into first line **16**) are as follows:

where:

$$Q_{AR} = C_{AR} \cdot Q_{TOT}$$

$$Q_{AIR} = (1 - C_{AR} - C_{O2})/(1 - C_{O2AIR}) \cdot Q_{TOT}$$

$$Q_{O2} = Q_{TOT} - Q_{AIR} - Q_{AR}$$

- $C_{AR}$ is the concentration of Ar in the mixture (here 50%),
- $C_{O2}$ is the concentration of $O_2$ in the mixture (here 21%), and
- $C_{O2AIR}$ is the concentration of $O_2$ in the medicinal air source (i.e. between 19.5 and 23.5 vol.%).

If $C_{O2AIR}$ is 21%, then for a total flow $Q_{TOT}$ of 10L/min, QAR=5L/min, $Q_{O2}$=1.33L/min and $Q_{AIR}$=3.67L/min. The gaseous flow travelling into first common line **40,** which is the sum of the flows in the second and third lines **26** ($Q_{O2}$), **36** (QAR), is such that the oxygen concentration in said line **40** is: $Q_{O2}$/($Q_{AIR}$ + $Q_{O2}$) = 21 vol. %.

However, if $C_{O2AIR}$ is 19.5%, $Q_{AIR}$ =3.6L/min and $Q_{O2}$=1.4L/min, then the oxygen concentration in line **40** is of 21.8 vol.%.

Conversely, if $C_{O2AIR}$ is 23.5%, $Q_{AIR}$ =3.8L/min and $Q_{O2}$=1.2L/min, then the oxygen concentration in line **40** is of 19.5%.

As can be seen, taking into account the concentration of oxygen $C_{O2AIR}$ composing the medicinal air delivered by the first source **10a,** impacts the flow traveling into the downstream portion **26** of the second line **23** (i.e. medical $O_2$ from second source **20a**) so that the overall oxygen concentration of the flow travelling into admission line **50** is at 21 vol.%.

The total flow $Q_{TOT}$ could range between 0 to about 150 L/min (i.e. 10 L/min is an example). Consequently, the first, second and third proportional valves **14, 24, 34** are sized in a way they can also accommodate such flow ranges.

Hence, according to the present invention, for correctly operating mixer **1** delivering a precise mixture to a patient **8,** it is mandatory to detect, in real time, whether the different elements, especially the mass flow sensors, operate correctly, for thereby preventing a delivery of a hypoxic mixture or a wrong gaseous mixture that does not fit with the one set by the user.

Indeed, the flow travelling into first common line **40** is the sum of the flows coming from the second line **26** (i.e. medical $O_2$), measured by the second mass flow sensor **25,** and the flow traveling into the third line **36** (i.e. therapeutic gas), measured by the third mass flow sensor **35.**

The ratio of these 2 gases yields to a specific lookup table determined by control unit **70** so that the fourth mass flow sensor **41** is correctly configured.

It should be clear at this stage that the mass flow sensor **41** should measure the sum of mass flow sensor **25** and mass flow sensor **35** measurement. Fourth mass flow sensor **41** therefore performs a "checksum" which ensures the integrity of the measurements.

Similarly, the flow traveling into admission line **50** is the sum of the flow coming from common line **40** (mixture composed of the therapeutic gas and medical oxygen), measured by fourth mass flow sensor **41,** and the flow coming from first line **16** (medicinal air), measured by first mass flow sensor **15.**

Here again, the ratio of these 2 gases yields to a specific lookup table determined by control unit **70** so that the fifth mass flow sensor **52** is correctly configured. The fifth mass flow sensor **52** should measure the sum of the first mass flow sensor **15** and the fourth mass flow sensor **41** measurement. Fifth mass flow sensor **52** therefore also performs a "checksum" which ensures the integrity of the measurements.

As all the mass flow sensors of mixer **1** are functioning properly, e.g. giving an accurate measurement, it can be determined the concentration of the therapeutic gas in the mixture, which is basically third mass-flow sensor **35** measurement over the total flow measured by fifth mass-flow sensor **52**. It can be further confirmed that the oxygen concentration in the mixture is of about 21%, as measured by oxygen sensor **51.**

As detailed above, the pre-use verification sequence ensures that all the mass-flow sensors of mixer **1** work properly and that the oxygen sensor **51** is fully calibrated.

During operation, the fourth and fifth mass flow sensors **41, 52** performs a real-time checksum thereby ensuring the integrity of the gas mixture.

In case of a sudden failure of one of the mass-flow sensors, for instance the second mass-flow sensor **25,** it can be determined within a few milliseconds that the checksum performed by fourth mass flow sensor **41** is not valid anymore and mixer **1** can act accordingly by warning the user and closing the third proportional valve **34** to stop the delivery of the hypoxic therapeutic gas.

Likewise, an oxygen concentration falling below a minimum threshold of 19.5% in the medicinal air, would be detected by the oxygen sensor **51** and the mixer **1** would interrupt the therapy and warn the user.

It should be noted the importance of having the oxygen sensor **51** placed in the admission line **50,** i.e. upstream of the vessel **53.** Indeed, the vessel **53** is a reserve of gas at the right oxygen concentration, e.g. 21%: if the oxygen concentration measured by oxygen sensor **51** drops below 19.5%, which is typically detected within 5s with Zirconia based oxygen sensors, only a fraction of the internal volume of vessel **53** will be replaced by the "hypoxic" concentration but the overall oxygen concentration of the mixture exiting vessel **53** will be mitigated by the large amount of gas in vessel **53** at the right concentration. Should the oxygen sensor be placed downstream of vessel **53,** the patient would actually inhale an "hypoxic" mixture before the detection occurs, which is not desirable.

The example illustrating the operation of mixer **1,** e.g. a mixture composed of 50% Ar, 21% $O_2$ and balance air (i.e. mainly $N_2$), allows in practice the setting of a concentration of oxygen ranging from 21 to 50 vol.%, depending on the need of the patient **8** to be treated.

Such a broad range of concentration of $O_2$ allows using the mixer **1** of the present invention for providing gas to various populations of patients, including those that are sedated and under mechanical ventilation.

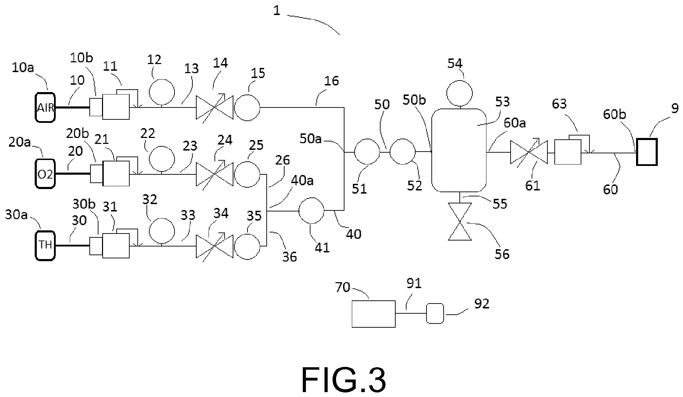

Next, Figure 2 shows a mixer **1** according to the present invention, such as the mixer **1** of Figure 3, connected to a mechanical ventilator **9** for feeding said ventilator **9** with a desired gas mixture and afterwards a patient **8** that is mechanically-ventilated.

The mechanical ventilator **9** delivers a life-support ventilation to the patient **8** via a breathing circuit **80,** such as gas hose(s) or the like, fluidly connected to said ventilator **9.**

As in Figure 1, the mixer **1** is connected, upstream, to the first, second and third gas sources **10a, 20a, 30a** via respective first, second and third inlet line **10, 20, 30,** and, downstream, to an inlet port **92b** of ventilator **9** so that the different gases coming from the first, second and third gas sources **10a, 20a, 30a** can be mixed inside the vessel **53** contained in the mixer **1** in desired proportions, as above explained, and then delivered to the ventilator **9** as a suitable gas mixture.

In one embodiment, inlet port **92b** can be, on a conventional and unmodified ventilator, the air inlet of the ventilator **9,** that normally only receives medicinal air at pressure greater than 2 bar abs, whereas in another embodiment, the ventilator **9** has been modified and comprises an inlet port **92b** that is specific and can only be connected to a mixer **1** according to the present invention, especially the mixer **1** of Figure 3.

Figure 3 shows another embodiment of a mixer **1** according to the present invention that is very similar to the one of Figure 1, except that the pressure sensor **62** of the mixer **1** of Figure 1 has been replaced by a fourth pressure regulator **63.** The fourth pressure regulator **63** maintains a constant gas pressure in the delivery line **60,** for instance a pressure of about 2 bar abs, to fit with the requirements of ventilator **9** that is fed by delivery line **60.**

As shown in Figure 2, the ventilator **9** further exhibits an additional port **90b** that can only be connected via a supply line **90** to a source **90a** of medical oxygen that can be the same source as the second gas source **20a,** in particular when said second gas source **20a** is an oxygen plug arranged on the wall of a hospital, or another oxygen source.

In operation, the ventilator **9** delivers a mechanical ventilation to the patient **8** at an oxygen concentration set by the user via a setting interface **92.** The oxygen concentration can vary, depending on the patient needs, between 21 vol.% (only port **92b** is used) and 50 vol.% (use of additional port **90b** and gas source **90a**), whereas the argon concentration in the mixture has to be kept constant, at 50 vol.%, i.e., regardless of the oxygen concentration set by the user.

Thus, when the user sets an oxygen concentration to be delivered to the patient **8:**

A) set to 21% of $O_2$: only port **92b** is opened and the mixer **1** delivers a gas mixture containing 50% Ar, 21% $O_2$ and 29% $N_2$ (vol. %),

B) set to 50% of $O_2$: the mixer **1** only provides a mixture of 21% $O_2$ and 79% Ar (no air is added as first valve **14** is closed). Supplemental oxygen is added via port **90b** of the ventilator **9,** thereby diluting the Ar concentration and obtaining a final mixture of 50% Ar and 50% $O_2$ (vol. %).

C) set to between 21% and 50% (vol. %), the mixer **1** adjusts the ratio of the different gases by controlling the first, second and third valves **14, 24, 34.** Additional $O_2$ can be provided, if necessary, from additional $O_2$ source **90a.**

This means that, in operation, the mixer **1** and ventilator **9** cooperate together. To this end, ventilator **9** communicates to mixer **1,** via line **91** (connected to control unit **70** as shown in Fig. 3) the oxygen concentration to be delivered to the patient **8** as set by the user via the interface **92.** The mixer **1** then adjusts the ratio of the gases so that a proper mixture is delivered to the ventilator **9.**

Precisely controlling the concentrations of the gas mixture generated by the mixer **1,** especially the oxygen concentration, is very important for ensuring a constant oxygen concentration of minimum 21% in the gas mixture delivered to the patient while keeping adequate the concentration of the therapeutic gas.

The present invention concerns a mixer **1** conceived for delivering a precisely controlled gas mixture, having the ability of monitoring in real-time the gas concentrations in said mixture and further of assessing during pre-use verification and operation, the status of its components to ensure increased safety for the patient inhaling the gas mixture delivered by said mixer **1.**

**Claims**

1. A gas mixer (1) comprising:

    - a mixing vessel (53),
    - a first line (10) for providing a first gas, a second line (20) for providing a second gas and a third line (30) for providing a third gas, said first, second and third lines (10, 20, 30) being in fluid communication with the mixing vessel (53) for providing said first, second and third gases to said mixing vessel (53) and obtaining a gas mixture in said mixing vessel (53), and
    - a delivery line (60) in fluid communication with the mixing vessel (53) for recovering at least a part of the gas mixture contained in the mixing vessel (53), and wherein:
    - the first line (10) comprises a first pressure regulator (11), a first pressure sensor (12) and a first mass flow controller (14, 15) comprising a first proportional valve (14) and a first mass flow sensor (15),
    - the second line (20) comprises a second pressure regulator (21), a second pressure sensor (22) and a second mass flow controller (24, 25) comprising a second proportional valve (24) and a second mass flow sensor (25),
    - the third line (30) comprises a third pressure regulator (31), a third pressure sensor (32) and a third mass flow controller (34, 35) comprising a third proportional valve (34) and a third mass flow sensor (35),
    - the first, the second and the third lines (10, 20, 30) are in fluid communication with the mixing vessel (53) via an admission line (50), and
    - an oxygen sensor (51) is arranged on the admission line (50).

2. A gas mixer (1) according to claim 1, wherein the admission line (50) is in fluid communication with the first, second and third lines (10, 20, 30) and with the mixing vessel (53).

3. A gas mixer (1) according to claim 1, wherein the admission line (50) is in fluid communication with the second and third lines (10, 20, 30) via a first common line (40).

4. A gas mixer (1) according to claim 3, wherein a fourth mass flow sensor (41) is arranged on the first common line (40).

5. A gas mixer (1) according to claim 1, wherein a fifth mass flow sensor (52) is arranged on the admission line (50) between the oxygen sensor (51) and the mixing vessel (53).

6. A gas mixer (1) according to claim 1, wherein the mixing vessel (53) is further fluidly connected to a delivery line (60) for withdrawing and conveying at least a part of the gaseous mixture contained in said mixing vessel (53).

7. A gas mixer (1) according to claim 6, wherein the delivery line (60) comprises a fourth proportional valve (61).

8. A gas mixer (1) according to claim 7, wherein the delivery line (60) further comprises a fourth pressure sensor (62) arranged downstream of the fourth proportional valve (61).

9. A gas mixer (1) according to claim 7, wherein the delivery line (60) further comprises a fourth pressure regulator (63) arranged downstream of the fourth proportional valve (61).

10. A gas mixer (1) according to claim 1, further comprising a control unit (70).

11. A gas mixer (1) according to claim 1, wherein the first line (10) is fluidly connected to a first gas source (10a), the second line (20) is fluidly connected to a second gas source (20a) and the third line (30) is fluidly connected to a third gas source (30a).

12. A gas mixer (1) according to claim 11, wherein the first gas source (10a) is air.

13. A gas mixer (1) according to claim 11, wherein the second gas source (20a) is an oxygen gas.

14. A gas mixer (1) according to claim 11, wherein the third gas source (30a) is a therapeutically-effective gas.

15. A gas mixer (1) according to claim 14, wherein the therapeutically-effective gas is chosen among $N_2O$, argon, xenon, helium or nitrogen.

16. A gas mixer (1) according to claim 10, further comprising a setting interface (92) connected to the control unit (70).

17. A gas mixer (1) according to claim 10, wherein the control unit (70) is configured for processing pressure signals received from the pressure sensors (12, 22, 32) and from the mass flow sensors (15, 25, 35).

18. A gas mixer (1) according to claim 10, wherein the control unit (70) is configured for processing oxygen concentration signals received from the oxygen sensor (51).

19. A gas mixer (1) according to claim 10, wherein the control unit (70) is configured for controlling the proportional valves (14, 24, 34, 61).

20. Mechanical ventilation system (1, 9) comprising a gas mixer (1) according to claim 1 in fluid communication with a mechanical ventilator (9) for providing a gas mixture to said mechanical ventilator (9).


**Patentansprüche**

1. Gasmischer (1), umfassend:

   - einen Mischbehälter (53),
   - eine erste Leitung (10) zum Bereitstellen eines ersten Gases, eine zweite Leitung (20) zum Bereitstellen eines zweiten Gases und eine dritte Leitung (30) zum Bereitstellen eines dritten Gases, wobei die erste, zweite und dritte Leitung (10, 20, 30) mit dem Mischbehälter (53) in Fluidkommunikation stehen, um das erste, zweite und dritte Gas an den Mischbehälter (53) bereitzustellen und ein Gasgemisch in dem Mischbehälter (53) zu erhalten, und
   - eine Förderleitung (60) in Fluidkommunikation mit dem Mischbehälter (53) zum Rückgewinnen mindestens eines Teils des in dem Mischbehälter (53) enthaltenen Gasgemisches,

   und wobei:

   - die erste Leitung (10) einen ersten Druckregulator (11), einen ersten Drucksensor (12) und einen ersten Massendurchflussregler (14, 15), der ein erstes Proportionalventil (14) und einen ersten Massendurchflusssensor (15) umfasst, umfasst,
   - die zweite Leitung (20) einen zweiten Druckregulator (21), einen zweiten Drucksensor (22) und einen zweiten Massendurchflussregler (24, 25), der ein zweites Proportionalventil (24) und einen zweiten Massendurchflusssensor (25) umfasst, umfasst,
   - die dritte Leitung (30) einen dritten Druckregulator (31), einen dritten Drucksensor (32) und einen dritten Massendurchflussregler (34, 35), der ein drittes Proportionalventil (34) und einen dritten Massendurchflusssensor (35) umfasst, umfasst,
   - die erste, die zweite und die dritte Leitung (10, 20, 30) über eine Zustromleitung (50) mit dem Mischbehälter (53) in Fluidkommunikation stehen, und
   - ein Sauerstoffsensor (51) auf der Zustromleitung (50) angeordnet ist.

2. Gasmischer (1) nach Anspruch 1, wobei die Zustromleitung (50) mit der ersten, zweiten und dritten Leitung (10, 20, 30) und mit dem Mischbehälter (53) in Fluidkommunikation steht.

3. Gasmischer (1) nach Anspruch 1, wobei die Zustromleitung (50) über eine erste gemeinsame Leitung (40) mit der zweiten und dritten Leitung (20, 30) in Fluidkommunikation steht.

4. Gasmischer (1) nach Anspruch 3, wobei ein vierten Massendurchflusssensor (41) auf der ersten gemeinsamen Leitung (40) angeordnet ist.

5. Gasmischer (1) nach Anspruch 1, wobei ein fünfter Massendurchflusssensor (52) auf der Zustromleitung (50) zwischen dem Sauerstoffsensor (51) und dem Mischbehälter (53) angeordnet ist.

6. Gasmischer (1) nach Anspruch 1, wobei der Mischbehälter (53) weiter mit einer Förderleitung (60) zum Abziehen und Transportieren mindestens eines Teils des in dem Mischbehälter (53) enthaltenen Gasgemisches fluidisch verbunden ist.

7. Gasmischer (1) nach Anspruch 6, wobei die Förderleitung (60) ein viertes Proportionalventil (61) umfasst.

8. Gasmischer (1) nach Anspruch 7, wobei die Förderleitung (60) weiter einen vierten Drucksensor (62) umfasst, der dem vierten Proportionalventil (61) nachgeschaltet angeordnet ist.

9. Gasmischer (1) nach Anspruch 7, wobei die Förderleitung (60) weiter einen vierten Druckregulator (63) umfasst, der dem vierten Proportionalventil (61) nachgeschaltet angeordnet ist.

10. Gasmischer (1) nach Anspruch 1, weiter umfassend eine Steuereinheit (70).

11. Gasmischer (1) nach Anspruch 1, wobei die erste Leitung (10) mit einer ersten Gasquelle (10a) fluidisch verbunden ist, die zweite Leitung (20) mit einer zweiten Gasquelle (20a) fluidisch verbunden ist und die dritte Leitung (30) mit einer dritten Gasquelle (30a) fluidisch verbunden ist.

12. Gasmischer (1) nach Anspruch 11, wobei die erste Gasquelle (10a) Luft ist.

13. Gasmischer (1) nach Anspruch 11, wobei die zweite Gasquelle (20a) ein Sauerstoffgas ist.

14. Gasmischer (1) nach Anspruch 11, wobei die dritte Gasquelle (30a) ein therapeutisch wirksames Gas ist.

15. Gasmischer (1) nach Anspruch 14, wobei das therapeutisch wirksame Gas aus $N_2O$, Argon, Xenon, Helium oder Stickstoff ausgewählt ist.

16. Gasmischer (1) nach Anspruch 10, weiter umfassend eine mit der Steuereinheit (70) verbundene Einstellungsbenutzeroberfläche (92).

17. Gasmischer (1) nach Anspruch 10, wobei die Steuereinheit (70) zum Verarbeiten von Drucksignalen, die von den Drucksensoren (12, 22, 32) und von den Massendurchflusssensoren (15, 25, 35) erhalten werden, konfiguriert ist.

18. Gasmischer (1) nach Anspruch 10, wobei die Steuereinheit (70) zum Verarbeiten von Sauerstoffkonzentrationssignalen, die von dem Sauerstoffsensor (51) erhalten werden, konfiguriert ist.

19. Gasmischer (1) nach Anspruch 10, wobei die Steuereinheit (70) zum Steuern der Proportionalventile (14, 24, 34, 61) konfiguriert ist.

20. Mechanisches Beatmungssystem (1, 9), umfassend einen Gasmischer (1) nach Anspruch 1 in Fluidkommunikation mit einem mechanischen Beatmungsgerät (9) zum Bereitstellen eines Gasgemisches an das mechanische Beatmungsgerät (9).

**Revendications**

1. Mélangeur de gaz (1) comprenant :

    - un récipient de mélange (53),
    - une première conduite (10) pour fournir un premier gaz, une deuxième conduite (20) pour fournir un deuxième gaz et une troisième conduite (30) pour fournir un troisième gaz, lesdites première, deuxième et troisième conduites (10, 20, 30) étant en communication fluidique avec le récipient de mélange (53) pour fournir lesdits premier, deuxième et troisième gaz audit récipient de mélange (53) et obtenir un mélange de gaz dans ledit récipient de mélange (53), et
    - une conduite de délivrance (60) en communication fluidique avec le récipient de mélange (53) pour récupérer au moins une partie du mélange de gaz contenu dans le récipient de mélange (53)

et dans lequel :

    - les premières conduites (10) comprennent un premier régulateur de pression (11), un premier capteur de pression (12) et une première unité de commande de débit massique (14, 15) comprenant une première vanne proportionnelle (14) et un premier capteur de débit massique (15),

- la deuxième conduite (20) comprend un deuxième régulateur de pression (21), un deuxième capteur de pression (22) et une deuxième unité de commande de débit massique (24, 25) comprenant une deuxième vanne proportionnelle (24) et un deuxième capteur de débit massique (25),

- la troisième conduite (30) comprend un troisième régulateur de pression (31), un troisième capteur de pression (32) et une troisième unité de commande de débit massique (34, 35) comprenant une troisième vanne proportionnelle (34) et un troisième capteur de débit massique (35),

- les première, deuxième et troisième conduites (10, 20, 30) sont en communication fluidique avec le récipient de mélange (53) via une conduite d'admission (50), et

- un capteur d'oxygène (51) est agencé sur la conduite d'admission (50).

2. Mélangeur de gaz (1) selon la revendication 1, dans lequel la conduite d'admission (50) est en communication fluidique avec les première, deuxième et troisième conduites (10, 20, 30) et avec le récipient de mélange (53).

3. Mélangeur de gaz (1) selon la revendication 1, dans lequel la conduite d'admission (50) est en communication fluidique avec les deuxième et troisième conduites (20, 30) via une première conduite commune (40).

4. Mélangeur de gaz (1) selon la revendication 3, dans lequel un quatrième capteur de débit massique (41) est agencé sur la première conduite commune (40).

5. Mélangeur de gaz (1) selon la revendication 1, dans lequel un cinquième capteur de débit massique (52) est agencé sur la conduite d'admission (50) entre le capteur d'oxygène (51) et le récipient de mélange (53).

6. Mélangeur de gaz (1) selon la revendication 1, dans lequel le récipient de mélange (53) est en outre fluidiquement relié à une conduite de délivrance (60) pour retirer et transporter au moins une partie du mélange gazeux contenu dans ledit récipient de mélange (53).

7. Mélangeur de gaz (1) selon la revendication 6, dans lequel la conduite de délivrance (60) comprend une quatrième vanne proportionnelle (61).

8. Mélangeur de gaz (1) selon la revendication 7, dans lequel la conduite de délivrance (60) comprend en outre un quatrième capteur de pression (62) agencé en aval de la quatrième vanne proportionnelle (61).

9. Mélangeur de gaz (1) selon la revendication 7, dans lequel la conduite de délivrance (60) comprend en outre un quatrième régulateur de pression (63) agencé en aval de la quatrième vanne proportionnelle (61).

10. Mélangeur de gaz (1) selon la revendication 1, comprenant en outre une unité de commande (70).

11. Mélangeur de gaz (1) selon la revendication 1, dans lequel la première conduite (10) est fluidiquement reliée à une première source de gaz (10a), la deuxième conduite (20) est fluidiquement reliée à une deuxième source de gaz (20a) et la troisième conduite (30) est fluidiquement reliée à une troisième source de gaz (30a).

12. Mélangeur de gaz (1) selon la revendication 11, dans lequel la première source de gaz (10a) est de l'air.

13. Mélangeur de gaz (1) selon la revendication 11, dans lequel la seconde source de gaz (20a) est un gaz d'oxygène.

14. Mélangeur de gaz (1) selon la revendication 11, dans lequel la troisième source de gaz (30a) est un gaz efficace d'un point de vue thérapeutique.

15. Mélangeur de gaz (1) selon la revendication 14, dans lequel le gaz efficace d'un point de vue thérapeutique est choisi parmi le $N_2O$, l'argon, le xénon, l'hélium ou l'azote.

16. Mélangeur de gaz (1) selon la revendication 10, comprenant en outre une interface de paramétrage (92) reliée à l'unité de commande (70).

17. Mélangeur de gaz (1) selon la revendication 10, dans lequel l'unité de commande (70) est configurée pour traiter des signaux de pression reçus en provenance des capteurs de pression (12, 22, 32) et en provenance des capteurs de débit massique (15, 25, 35).

**18.** Mélangeur de gaz (1) selon la revendication 10, dans lequel l'unité de commande (70) est configurée pour traiter des signaux de concentration d'oxygène reçus en provenance du capteur d'oxygène (51).

**19.** Mélangeur de gaz (1) selon la revendication 10, dans lequel l'unité de commande (70) est configurée pour commander les vannes proportionnelles (14, 24, 34, 61).

**20.** Système de ventilation mécanique (1, 9) comprenant un mélangeur de gaz (1) selon la revendication 1 en communication fluidique avec un ventilateur mécanique (9) pour fournir un mélange de gaz audit ventilateur mécanique (9).

EP 3 556 420 B1

FIG.1

FIG.2

14

FIG.3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080121233 A
- US 20090205661 A

- EP 2489392 A